# EUROPEAN PATENT SPECIFICATION

(11) **EP 0 601 052 B1**
(45) Date of publication and mention of the grant of the patent: **16.10.1996**
(21) Application number: 92918792.0
(22) Date of filing: 19.08.1992
(51) Int. Cl.: A61K 39/395, A61K 38/00, A61K 38/21

(54) **THERAPEUTIC METHOD FOR THE TREATMENT OF IDDM**
THERAPEUTISCHES VERFAHREN ZUR BEHANDLUNG VON IDDM
PROCEDE THERAPEUTIQUE SERVANT A TRAITER LE DIABETE SUCRE INSULINODEPENDANT

(30) Priority: 30.08.1991 US 752309
(43) Date of publication of application: 15.06.1994
(73) Proprietor: GENENTECH, INC., South San Francisco California 94080 (US)
(72) Inventor: STEWART, Timothy, Andrew, San Francisco, CA 94114 (US)
(74) Representative: Barz, Peter, Dr.
(86) International application number: US9207094
(87) International publication number: WO9304699

(56) References cited:
- EP-A- 0 304 291
- EP-A- 0 369 877
- DIABETIC MEDICINE, vol. 8, no. 6, July 1991; G. TOMS et al., pp. 547-550
- JOURNAL OF BIOLOGICAL REGULATORS & HOMEOSTATIC AGENTS, vol. 3, no. 2, April 1989, Milano (IT); A. BOUCHER, pp. 47-49

## Description

This invention relates to the prevention or amelioration of the onset or maturation of type I diabetes.

Type I diabetes (insulin-dependent diabetes mellitus, or IDDM) is well known clinically (Wyngaarden et al., Cecil Textbook of Medicine, 1988). It is the result of the failure to produce sufficient insulin and ultimately of the loss of the insulin producing beta islet cells of the pancreas. Hypoinsulinemia and hyperglucagonemia characterize IDDM, and patients readily develop ketosis as well as other severe and debilitating conditions. At the time of first clinical presentation, symptoms can be traced back for several days to a few weeks. While in most cases islet cell destruction had been going on for months and often years before the onset of symptoms, residual islet cells often remain in IDDM patients (34 out of 37 pancreases removed from IDDM patients had residual beta cells [Foulis et al., Lancet December 19, 1987, pp 1423-14271]). In some cases, preclinical IDDM can be detected by determining the patient's ability to adequately respond to a glucose challenge or assaying for the presence of circulating antibodies to islet cells or insulin. The peak onset of IDDM is 11 to 13 years although still substantial incidence occurs in later years. It is unusual for IDDM to begin after age 40.

Once IDDM is diagnosed a tight regimen of calorie regulation is introduced and the patient placed on insulin therapy. In many patients a rebound or "honeymoon" period follows initial treatment in which the disease is in remission and little or no insulin is required. Remission is due to a partial return of endogenous insulin secretion, which may last for several weeks or months and occasionally 1 to 2 years; ultimately, however, the disease recurs and insulin therapy is required permanently.

The etiology of IDDM is a matter of great debate. It appears to be multifactorial, including genetic predisposition and environmental influences. Strong associations have been identified between IDDM and specific HLAs coded by the major histocompatibility complex region located on the short arm of chromosome 6. The major high risk alleles are DR3 and DR4.

The environmental influences thought to be important include viruses, and several lines of evidence support this view. Certain diabetogenic or associated viruses (mumps, measles, rubella, encephalomyocarditis M, coxsackievirus B, and rheovirus) have been associated epidemiologically with the development of IDDM or can cause diabetes when inoculated into rodents. Diabetogenic viruses can directly infect B cells in culture. In addition, coxsackievirus B4 has been isolated from the pancreas of a boy with new-onset IDDM who died of severe ketoacidosis, and this virus produced diabetes in experimental animals together with viral antigens in the B cells of the infected animals. Foulis et al., (Id.) found that B cells of IDDM patients secreted alpha interferon, and concluded from this that chronic viral infection of B cells may underlie the pathogenesis of some cases of IDDM.

EP-A-304291 discloses the use of certain gamma interferon antagonists in the treatment of MHC class II associated autoimmune diseases such as juvenile insulin-dependent diabetes.

Autoimmunity may also play a role in IDDM. Up to 90% of patients with new-onset IDDM have titers of antibodies to islet cells. Both antibody induced and cell mediated immune phenomena may be involved in the pathogenesis of IDDM.

The relationship of the various factors involved in IDDM is unclear and may vary from patient to patient.

Treatment of IDDM by administration of exogenous insulin is unsatisfactory in many respects. Aside from the need for frequent injections it is difficult in practice to maintain euglycemia because of the varying insulin demands posed by changes in calorie intake and composition and because of variation in the source and type of insulin preparation, its delivery route, rate of absorption and the purity and characteristics of the insulin preparations. Attempts have been made to improve insulin homeostasis by continuous subcutaneous insulin infusion or by other sustained release devices, but substantial variations in blood glucose are commonly encountered. In addition, a major complication of exogenous insulin is hypoglycemia, a potentially life-threatening condition stemming from excessive insulin dosages.

While studies are underway to develop tissue implants capable of secreting insulin, it would be preferable to save the patient's own islet cells by interdicting the process of IDDM as it occurs. However, until now the uncertain and multiple etiology of IDDM has made the identification of the therapeutic target quite difficult.

Accordingly, it is an object of this invention to provide compositions for arresting, reversing or impeding the necrosis of islet cells during early onset and preclinical IDDM. It is another object of this invention to provide compositions for the treatment or prophylaxis of IDDM.

We have discovered that transgenic animals expressing islet-cell specific alpha interferon under the control of the insulin promoter develop insulin dependent diabetes and pancreatic inflammation. Accordingly, we have concluded that endogenous alpha interferon expression by islet cells is responsible for IDDM and the objects of this invention can be accomplished by administering a therapeutically effective amount of an alpha interferon antagonist to a preclinical or recent onset IDDM patient having at least some residual beta islet cell capability to secrete insulin.

Thus, the present invention relates to the use of an alpha interferon antagonist in the manufacture of a medicament for the treatment or prophylaxis of IDDM.

High local concentration of interferon gamma has been demonstrated to induce an autoimmune diabetes. This was achieved by creating a transgenic mouse which carried a fusion gene comprised of the human insulin promoter and the mouse gamma interferon gene, whereby the pancreatic islets expressed interferon gamma. This lead to local inflammation and destruction of the insulin producing cells of the islets. While this result was of significant scientific interest, its relevance to the early stages of IDDM was limited. Interferon gamma normally is expressed by immune cells (and not by islet cells), so it could only be present and play a role after inflammation has been initiated. That is, gamma interferon may have a role in the later stages of IDDM but cannot initiate it.

The search for a factor or an event that could initiate IDDM took into consideration several factors. First, as noted above, a significant number of studies suggest the involvement of viruses in the initiation of the disease. However, because these viruses do not cause an acute loss of the beta cells, the relationship is probably indirect. Second, because the inflammation is not pre-existing, the early stages of the disease must involve only the islets themselves. Third, previous work on transgenic mice in which the beta cells constitutively express the MHC antigens (Class I or Class II) demonstrated that the islets cannot initiate an autoimmune disease solely by self presentation of a self or a foreign antigen.

These considerations lead me to consider the possibility that a viral infection of the beta cells would lead to the synthesis and release of a protein that could stimulate resident macrophages (these cells are present in most normal tissues, including the pancreas). Once activated these macrophages could release a variety of lymphokines and cytokines and could initiate a destructive inflammation. One possible factor that could be made by epithelial cells such as beta cells in response to a viral infection is alpha interferon. Not only could this interferon be made by the islet cells in response to a stimulus, it can activate macrophages. While it was known that alpha interferon is produced by the islet cells of newly diagnosed IDDM patients (Fouls et al., Id.), there was no basis to conclude that the expression of this lymphokine was causative.

Accordingly, it was decided to engineer a transgenic mouse so that the beta cells would make alpha interferon. The constraints on the transgene were: 1) the promoter had to be such that the gene would be expressed in the beta cells (the preferable candidate was the insulin promoter and in fact it had been previously demonstrated that this promoter would direct expression of a heterologous gene to the beta cells in transgenic mice); 2) the alpha interferon would have to be active on mouse cells (there is considerable species specificity in the action of the interferons); 3) ideally, as it was uncertain whether the ultimate therapy would be directed against the receptor or against the ligand (alpha interferon) I wanted the alpha interferon to be human so that it would be convenient to screen effective antagonists. Thus, a fusion gene was generated in which the human insulin 5' non transcribed region, the human insulin first exon, first intron and that part of the human insulin second exond 5' to the translation start site was linked to the cDNA encoding human alpha interferon A/D Bg1 ll. This is a hybrid, the DNA for which is generated *in vitro,* between the human alpha interferon A and D genes using a common Bg1 ll restriction endonuclease site for the fusion. This hybrid human alpha interferon is active on mouse cells (unlike most human alpha interferons). The alpha interferon cDNA was followed by the poly A addition signal of the hepatitis surface antigen.

This Ins-Ifn.alpha fusion gene was injected into the pronuclei of single-cell mouse embryos and transgenic mice carrying the fusion gene were generated. The mice used to produce the eggs that were injected were an F1 hybrid between BALB/c females and DBA/2 males (BDF1). Thus the original transgenic mice were then backcrossed onto BALB/c and the transgenic progeny analyzed. These mice expressed the human alpha interferon gene (as determined by immunocytochemistry) and, associated with this expression, developed a severe pancreatic inflammation that was initially centered on the islets. This demonstrated that unrestrained production of alpha interferon by beta cells could lead to inflammation. However, these mice did not develop diabetes (hyperglycemia in a fed or fasted state). It is known that BALB/c mice are resistant to the induction of diabetes by either viral or chemical agents. Thus I considered the possibility that, although there was a significant pancreatic inflammation, diabetes did not develop in these mice either because the islets were resistant to those agents that kill islets in sensitive strains of mice (lymphokines, CTLs, superoxide radicals etc.) or because BALB/c mice are able to regenerate islets at a rate that keeps pace with the ongoing beta cell necrosis.

To address the role of alpha interferon the mice were backcrossed onto strains C57B1/6 and CD-1 in order to induce sensitivity to the induction of diabetes. Second generation backcross mice were generated and monitored for hyperglycemia. Thus far, two transgenic males (out of five transgenic males and four transgenic females) have become diabetic (one at four months of age and one at five months), and additional conversions are expected as the strain proceeds.

A variety of proteins have been expressed in the beta cells of transgenic mice such that the animals were immunologically tolerant toward these "foreign" antigens. In some cases these proteins are deleterious (Lo et al., Cell 53:159:168 [1988]), in some cases tolerance can be broken with deleterious consequences (Ohashi et al., Cell 65:305-317 [1991]; Oldstone et al., Cell 65:319-331 [1991]) and in at least one case tolerance could be broken with no deleterious consequences (hu CD4, Stewart et al., unpublished). Other proteins were expressed without causing diabetes, e.g. herpes gD, substance P, rat growth hormone, placental lactogen and NGF. In other instances nontolerance (and insulitis) was exhibited by transgenic mice in which the islets express viral antigens, or gamma interferon (Adams et al., Nature 325:223-228 [1987]; Sarvetnick et al., Cell 52:773-782 [1988]). In the case of alpha interferon as described here it is believed that the deleterious effect of alpha interferon is the result of its chronic and long term expression by beta cells (in the transgenic animals and in IDDM), whereas alpha interferon normally is only expressed transiently, for example in response to viral infections.

Interferons are secreted proteins produced by the cells of most vertebrates in response to viruses or other agents, which are characterized by their ability to induce an antiviral state in a variety of target cells (reviewed in Stewart, The Interferon System, 1979). Interferons have been shown to modulate the activity of T- and B-lymphocytes, natural killer cells, macrophages and other cells involved in immune response, and to regulate the growth of tumor cells and other proliferating cell types. Several types of interferons have been differentiated on the basis of cellular origin, and biochemical and antigenic properties. IFN-α and IFN-β (also known as the Type I interferons) represent the major interferons synthesized by leukocytes and fibroblasts, respectively, following treatment with viruses, double-stranded RNA or other inducers. IFN-γ is produced by T-lymphocytes that have been stimulated by mitogens or specific antigens.

Human IFN-β and IFN-γ are specified by unique genes (Taniguchi et al., Nature 285:547-549 [1980]; Derynck et al., Nature 285:542-547 [1980]; Goeddel et al., Nucleic Acids Res. 8:4057-4074 [1980b]). The gene for HuIFN-β lacks introns (Lawn et al., Proc. Natl. Acad. Sci. 78:5435-5439 [1981b]; Degrave et al., Gene 14:137-143 [1981]; Ohno et al., Proc. Natl. Acad. Sci. 78:5305-5309 1981]) and encodes a protein possessing 29 percent amino acid homology with HuIFN-αl, suggesting that IFN-α and IFN-β genes have evolved from a common ancestor (Taniguchi et al., Nature 285:547-549 [1980]). By contrast, the HuIFN-γ coding region is divided by three introns (Gray et al., Nature 298:859-863 [1982]), and exhibits extremely limited amino acid homology with HuIFN-α (Gray et al., Nature 295:503-508 [1982]; Epstein, Nature 295:453-454 [1982]). Interestingly, while only a single HuIFN-β gene has been unequivocally identified, bovine IFN-β is encoded by a family of five or more homologous, yet distinct genes (Leung et al., Biotechnology, 1984). Moreover, the size of the IFN-β gene family varies considerably among different mammalian species (Leung et al., Biotechnology, 1984).

Alpha interferon, for the purposes herein, includes all members of the alpha interferon family identified previously, as well as future members of the family which are encoded by nucleic acid capable of hybridizing under low stringency conditions to DNA encoding known members of the family and which possess at least one qualitative biological activity of an alpha interferon. Low stringency hybridization conditions are defined as follows: hybridization to filter-absorbed nucleic acid is performed in 5XSSC (1XSSC is 0.015 M NaCL, 0.15 M Nacl, 0.015 M sodium citrate), 5X Denhardt's solution (Denhardt, 1966), 0.1 percent sodium dodecyl sulfate (SDS), 0.1 percent sodium pyrophosphate, 50 µg/ml sonicated, denatured salmon sperm DNA and 10 percent sodium dextran sulfate, containing 20 percent formamide. After incubation at 42°C, the filter is washed at room temperature in 2XSSC containing 0.2 percent SDS.

Alpha interferon polypeptides identified previously fall into two major classes, I and II, each containing a plurality of discrete proteins (Baron et al., Critical Reviews in Biotechnology 10:179-190 [1990]; Nagata et al., Nature 287:401-408 [1980a]; Nagata et al., Nature 284:316-320; [1980b]; Streuli et al., Science 209:1343-1347 [1980]; Goeddel et al., Nature 287:411-416 [1980a]; Goeddel et al., Nature 290:20-26 [1981]; Lawn et al., Science 212:1159-1162 [1981a]; Ullrich et al., J. Mol. Biol. 156:467-486 [1982]; Weissmann et al., Phil. Trans. R. Soc. Lond. B299:7-28 [1982]; Lund et al., Proc. Natl. Acad. Sci. 81:2435-2439 [1984]; Capon et al., Mol. Cell. Biol. 5:768 [1985]). Alpha interferons include IFN-alphaK, IFN-alpha5, IFN-alphaA (IFN-alpha2), IFN-alphaD (IFN-alpha1), IFN-alphaH1, IFN-alphaB2, IFN-alphaB, IFN-alpha4b, IFN-alpha6, IFN-alphaC1, IFN-alphaC, IFN-alphaL, IFN-alphaJ1, IFN-alphaJ2, IFN-alpha74, IFN-alphaI, IFN-alphaF, IFN-alphaWA, IFN-alphaGx-1, IFN-alpha76 (IFN-alpha4a), IFN-alpha88 and alleles thereof.

An alpha interferon antagonist is defined to be any substance that is capable of interfering with a biological activity of alpha interferon *in vivo.* It is not necessary that the antagonist completely neutralize the alpha interferon activity, but only that it do so to a degree sufficient to exert an IDDM therapeutic activity *in vivo.* Alpha interferon is known to possess a plurality of biological activities. The antagonists for use herein will reduce, inhibit or neutralize any one or more of these activities. Ordinarily the antagonist will interfere with at least one (and preferably all) of the antiviral, antiproliferative or the immunomodulatory activity of alpha interferon.

The antagonists generally are selected from several categories: A soluble form of the alpha interferon receptor, anti-alpha interferon receptor antibodies which block alpha interferon from binding or properly interacting with its receptor, antibodies capable of binding and neutralizing alpha interferon itself, interferon fragments or other amino acid sequence variants of alpha interferon that antagonize alpha interferon activity, and non-interferon polypeptides which compete with alpha interferon for receptor binding sites but which do not themselves demonstrate substantial alpha interferon activity. However, the invention is not limited to these categories since it is quite conceivable that nonpeptidyl or other polypeptide alpha interferon antagonists will be developed in the future.

Anti-alpha interferon antagonist antibodies are well known *per se* (Tsukui et al., Microbiol. Immunol. 30:1129-39 [1986]; Duarte et al., Interferon-Biotechnol. 4:221-232 [1987]; Barasoain et al., J. Immunol. 143:507-512 [1989]; Exley et al., J. Gen. Virol. 65:2277-80 [1984]; Shearer et al., J. Immunol. 133:3096-101 [1984]; Alkan et al., Ciba Geigy Foundation Symposium 119:264-78 [1986]; Noll et al., Biomed. Biochim. Acta 48:165-176 [1989]; Hertzog et al., J. Interferon Res. 10(Suppl. 1) [1990]; Overall et al., J. Immunol. Methods 119:27-33 [1989]; Kawada et al., Immunology 56:489-495 [1985]; Kontsek et al., J. Interferon Res. (special issue) 73-82 [1991]; Adolf et al., EP 119,476; Wisniewski et al., DD 277087; Hauptmann et al., US 4,917,887; and Ebrain et al., GB 2,195,342). The antibody preferably is selected so as to neutralize the biological activity of the subtype(s) of alpha interferon produced by beta islet cells (see Lydon et al., Biochemistry 24:4131-4141 [1985]) for an example of subtype specific neutralizing antibody). The subtype expressed by IDDM beta islet cells is readily determined by screening a panel of beta islet cells from patients with pre-clinical IDDM or patients during the "honeymoon" phase of IDDM therapy. Alternatively, a biopsy of the patient to be treated can be taken in order to determine the patient's subtype. The assay is a straight-forward matter which can be accomplished by numerous methods. For example, cDNA can be prepared from freshly excised pancreas samples and amplified by PCR using a bank of primers specific for each alpha interferon subtype. Alternatively, the subtype can be determined by immunohistochemical analysis using subtype specific antibodies. In addition, the antibody should be capable of neutralizing the antiviral (for example Nisbet et al., Biochem. Int. 11:301-309 [1985]) and antiproliferative (for example Cebrain et al., Infra or Evinger et al., Methods Enzymol. 79:362-368 [1981]) activity of alpha interferon, most preferably the immunomodulatory activity alone. The immunomodulatory activity to be neutralized preferably is IFN boosted NK activity (Cebrian et al., J. Immunol. 138:484-490 [1987]; Lee et al., Cancer Res. 42:1312 [1982]) or augmentation of monocytes or macophages function, e.g. protease production (Jones et al., J. Interferon Res. 2:377 [1982]).

The alpha interferon neutralizing antibodies should be capable of binding to the naturally occurring interferon produced by islet cells. Accordingly, the system used to generate and select the antibody will be targeted to the natural product purified from cell lines, body fluids or primary cultures of the animal species to be treated for IDDM. The antibody is desirably selected so that it binds and neutralizes a naturally-occurring form of the interferon, but does not neutralize (or neutralizes to a lesser degree) a selected recombinant form of alpha interferon such as the interferon alpha2 variants now being marketed by the Schering-Plough Corporation and Hoffmann-La Roche, Inc. Ordinarily, this will mean selecting antibodies that are capable of neutralizing mammalian cell products, but not the products of recombinant lower organisms such as E.Coli (Tsukui et al., Microbiol. Immunol. 30:1271-9 [1986]). The procedures to be used in selecting the desired neutralizing antibodies are conventional. Suitable methods are disclosed by the citations above. In general, one simply determines the dose related ability of the antibody to interfere with and suppress the biological activity of alpha interferon in the otherwise conventional target assay. Those antibodies which require the lowest dose to effect detectable alpha interferon inhibition, and in particular those that are capable of acting to do so even after the interferon has been introduced into the assay system, are selected for use herein.

Another suitable group of alpha interferon antagonists are antibodies capable of neutralizing the activity of alpha interferon receptor polypeptides. At this point at least two such receptor polypeptides have been identified (Revel et al., EP 369,877; Mogensen et al., WO 91/05862; and Colamonici et al., Proc. Nat. Acad. Sci. 87:7230-7234 [1990]). It is as yet unclear whether the proteins described in these citations represent coordinately acting proteins or whether the proteins act independently. Apparently, alpha interferon and beta both recognize the same receptor structure, while gamma interferon recognizes another (Colamonici et al., Id.). Colamonici et al., Id. report that the alpha interferon receptor structure contains two polypeptide chains, one of which, the alpha chain, can be immunoprecipitated by antibodies. This protein, at about 110 Kd, may be the 95-100 Kd protein putatively identified by Mogensen et al., Id. as the alpha interferon receptor. For the purposes herein, antagonist antibodies are those raised against or capable of binding any one of the proteins described by Revel et al., Mogensen et al. or Colamonici et al. and which are able to compete with or otherwise antagonize alpha interferon. Ideally, the antibodies will be capable of displacing alpha interferon from the receptor binding site and will have a greater affinity for the receptor than does alpha interferon. It is not necessary that the antibodies bind to the receptor binding site for alpha interferon, only that they be capable of interfering with the interaction of the site with alpha interferon, as for example they may do by binding only in the vicinity of the site which is then sequestered by the steric bulk of the antibody. It also is not necessary that they have greater capacity than alpha interferon since any deficiencies in this regard can be remedied by elevating the dosage.

The anti-receptor neutralizing antibodies are identified in the same fashion as anti-alpha interferon antibodies, i.e., by their ability to inhibit or interfere with the biological activity of alpha interferon in otherwise conventional bioassays for alpha interferon. In the case of anti-receptor antibodies one should also screen for the ability of the antibody to act *per se* as an agonist for alpha interferon. Agonist antibodies are those which cross-link or aggregate alpha interferon receptors and, in doing so, mimic the effect of alpha interferon. Obviously, they are not desirable for use in the invention here, although they clearly are useful in place of alpha interferon.

Antagonist antibodies in general will be prepared as monovalent forms, i.e., they are only capable of binding to a single receptor at a time. Structurally, such antibodies will have only a single heavy/light chain arm, as for example in the case of Fab or Fab' fragments. They are prepared in conventional fashion, either by recombinant expression of truncated heavy chains together with light chains or by preparation of intact antibody followed by *in vitro* digestion with appropriate proteases to obtain the univalent antibody.

The anti-alpha interferon or receptor antibodies can be of any antibody class or subclass, including IgG, IgM, IgA or IgD. They may be polyclonal, monoclonal, or mixtures of monoclonal antibodies. Preferably the antibodies are IgG and are of subclasses that do not activate complement (or are mutagenized to the same effect). The antibodies include amino acid sequence or other covalent modifications of native antibodies, including heterobifunctional antibodies, antibody fragments such as Fab, Fab', and (Fab')2, single chain antibodies (in which the heavy and light chain are joined by an exogenous polypeptide), chimeric antibodies (interspecies, or interclass or subtype), CDR-grafted interspecies antibodies and polyethylene glycol-substituted or otherwise covalently modified antibodies. Heterobifunctional antibodies include those in which one arm of the antibody is capable of binding to and neutralizing alpha interferon or its receptor while the other arm binds to a different predetermined epitope or antigen such as different interferon or interferon receptor epitopes, or to insulin or an islet cell surface antigen. When used in human therapy, the antibodies should be human or CDR grafted into human antibody sequence in order to minimize immune responses to the antibody.

The alpha interferon receptor polypeptides which are capable of binding to alpha interferon also are useful as alpha interferon antagonists. These are used in substantially the same fashion as alpha interferon neutralizing antibodies. Preferably, such receptor polypeptides are modified in the same fashion as other receptors previously developed for therapeutic use, including solubization on for example by deletion or inactivation of the transmembrane domain plus, optionally, substituting a polypeptide with a long half life for the transmembrane and cytoplasmic region of the receptor protein substantially as shown in EP WO 314,317, WO 91/08298, or WO 90/06953.

Another antagonist class for use herein includes substances which down-regulate the expression of alpha interferon in tissues *in vivo.*

Still further antagonist classes are described by Delcayre et al., EMBO Journal 10:919-926 [1991]. Delcayre et al. disclose that Epstein Barr virus/complement C3d receptor is an alpha interferon receptor, and that alpha interferon binding to Raji cells is inhibited by anti-CR2 antibodies, by peptides with the CR2 binding motif and partially by C3bi/C3d. Thus, the CR2 receptor or a polypeptide comprising its interferon binding motif can be employed in the same fashion as the interferon receptor polypeptides described above, including their use in the form of variants such as truncations or fusions with immunoglobulin constant domains. Polypeptides containing the C3d binding site sequence also can be employed as competitive inhibitors for alpha interferon.

Other antagonists include fragments or amino acid sequence variants of alpha interferon which competitively inhibit native interferon but which have less or no significant interferon biological activity. An example is the 92-99 motif polypeptide pIFNalpha described by Delcayre et al., Id.

The foregoing are only examples of substances that are believed to be suitable antagonists. It will be understood that other antagonists will be developed in the future, and that this invention also contemplates their use in the treatment of IDDM.

It is within the scope of this invention to use 1, 2 or more of each antagonist, whether from within or without the same class. In addition, the antagonists can be administered in combination with other therapeutic agents for IDDM, including immunorepressive agents such as azothioprin or cyclosporin, and insulin.

The antagonists are administered through exogenous sources or are generated in situ. In the latter, antagonist antibodies are generated by immunizing the subject against endogenous alpha interferon or its receptor. This is best accomplished by conjugating the interferon or its receptor to a highly immunogenic protein and immunizing by subcutaneous administration with an adjuvant (such as TNF) and carrier. However, it is preferable to treat IDDM by administration of exogenous antagonist.

The pharmaceutical formulation of each antagonist of course will depend upon the chemical and physical nature of the antagonist selected. On balance, the antagonist will be formulated in a sterile composition that is pharmaceutically acceptable, i.e., is isotonic and pure to at least 95% by weight of protein. Typical bulking agents such as sugar alcohols or inert protein (such as albumin) are employed for lyophilized compositions. Stabilizers such as antioxidants and chelating agents also are included if required. The antagonist will be placed in a hermetically sealed container which is generally sealed with an elastomeric stopper for providing sterile needle access.

The therapeutically effective dose of antagonist will depend upon the condition of the patient, the competitive capability of the antagonist, its circulating half life, and other parameters determinable by the ordinary clinician. Antibody to alpha interferon or soluble receptor is administered at a dose calculated to sequester substantially all of the patient's endogenous circulating and cell surface alpha interferon. This in general will be a larger dose than that which is used for anti-receptor antibody, but probably less than that needed for an alpha interferon competitive inhibitor.

The patients to be treated with antagonist are preclinical IDDM patients or those in recent onset IDDM. Patients are candidates for therapy in accord with this invention until such point as the patient's islet cells are no longer viable. It is desirable to administer antagonist as early as possible in the development of IDDM, and treatment will continue for so long as is necessary to preserve islet cells. The IDDM patient is treated until insulin monitoring demonstrates adequate islet response and other indicia of islet necrosis diminish (e.g., anti-insulin and anti-islet antibodies), after which the patient can be withdrawn from antagonist treatment for a trial period during which insulin response and the level of anti-islet antibodies are monitored for relapse.

The antagonist is administered to the patient by any conventional route, including intravenous, intraperitoneal, subcutaneous, intrapulmonary, intranasal or the like. Preferably, it is administered by intravenous infusion on a continuous basis.

## Claims

1. The use of an alpha interferon antagonist in the manufacture of a medicament for the treatment or prophylaxis of insulin dependent diabetes mellitus.

2. The use of claim 1 wherein the medicament is for administration to a patient having at least residual beta islet cell insulin-secretory function.

3. The use of claim 1 or 2 wherein the antagonist is an antibody capable of binding to an alpha interferon receptor polypeptide.

4. The use of claim 1 or 2 wherein the antagonist is an antibody capable of binding to and neutralizing a biological activity of an alpha interferon.

5. The use of claim 1 or 2 wherein the antagonist is an alpha interferon receptor polypeptide.

6. The use of claim 4 wherein the alpha interferon is selected from IFN-alphaK, IFN-alpha5, IFN-alphaA (IFN-alpha2), IFN-alphaD (IFN-alpha1), IFN-alphaH1, IFN-alphaB2, IFN-alphaB, IFN-alpha4b, IFN-alphaC, IFN-alphaL, IFN-alphaJ1, IFN-alphaJ2, IFN-alphaI, IFN-alphaF, IFN-alphaWA, IFN-alphaGx-1, IFN-alpha76, IFN-alpha88 and alleles thereof.

7. The use of claim 4 wherein the antibody is capable of binding and neutralizing more than one interferon.

8. The use of claim 4 wherein the biological activity is selected from antiproliferative and antiviral activity.

9. The use of claim 4 wherein the antibody is capable of binding and neutralizing an alpha interferon synthesized by a eukaryotic cell but is substantially not capable of neutralizing alpha interferon of the same subclass which had been synthesized in recombinant bacterial cell culture.

10. The use of claim 4 wherein the alpha interferon is a class I alpha interferon.

11. The use of claim 4 wherein the alpha interferon is a class II alpha interferon.

12. The use of claim 2 wherein the antagonist is administered to the patient during the rebound period.

13. The use of claim 2 wherein the antagonist is administered to the patient prior to the development of insulin dependence.

## Patentansprüche

1. Verwendung eines α-Interferon-Antagonisten bei der Herstellung eines Medikaments zur Behandlung oder Prophylaxe von Insulin-abhängiger Diabetes mellitus.

2. Verwendung nach Anspruch 1, worin das Medikament zur Verabreichung an einen Patienten bestimmt ist, der mindestens eine Insulin-sekretorische β-Inselzell-Restfunktion aufweist.

3. Verwendung nach Anspruch 1 oder 2, worin der Antagonist ein Antikörper ist, der imstande ist, an ein α-Interferon-Rezeptor-Polypeptid zu binden.

4. Verwendung nach Anspruch 1 oder 2, worin der Antagonist ein Antikörper ist, der imstande ist, an eine biologische Aktivität eines α-Interferons zu binden und diese zu neutralisieren.

5. Verwendung nach Anspruch 1 oder 2, worin der Antagonist ein α-Interferon-Rezeptor-Polypeptid ist.

6. Verwendung nach Anspruch 4, worin das α-Interferon ausgewählt ist aus IFN-αK, IFN-α5, IFN-αA (IFN-α2), IFN-αD (IFN-α1), IFN-αH1, IFN-αB2, IFN-αB, IFN-α4b, IFN-αC, IFN-αL, IFN-αJ1, IFN-αJ2, IFN-αI, IFN-αF, IFN-αWA, IFN-αGx-1, IFN-α76, IFN-α88 und Allelen davon.

7. Verwendung nach Anspruch 4, worin der Antikörper imstande ist, mehr als ein Interferon zu binden und zu neutralisieren.

8. Verwendung nach Anspruch 4, worin die biologische Aktivität aus antiproliferativer und antiviraler Aktivität ausgewählt ist.

9. Verwendung nach Anspruch 4, worin der Antikörper imstande ist, ein von einer eukaryotischen Zelle synthetisiertes α-Interferon zu binden und zu neutralisieren, aber im wesentlichen nicht imstande ist, α-Interferon derselben Subklasse zu neutralisieren, welches in rekombinanter Bakterienzellkultur synthetisiert worden ist.

10. Verwendung nach Anspruch 4, worin das α-Interferon ein Klasse I-α-Interferon ist.

11. Verwendung nach Anspruch 4, worin das α-Interferon ein Klasse II-α-Interferon ist.

12. Verwendung nach Anspruch 2, worin der Antagonist dem Patienten während der Rebound-Periode verabreicht wird.

13. Verwendung nach Anspruch 2, worin der Antagonist dem Patienten vor der Entwicklung von Insulinabhängigkeit verabreicht wird.

## Revendications

1. Utilisation d'un antagoniste d'interféron alpha dans la fabrication d'un médicament pour le traitement ou la prophylaxie du diabète sucré insulinodépendant.

2. Utilisation suivant la revendication 1, dans lequel le médicament est destiné à l'administration à un patient ayant au moins une fonction insulino-sécrétoire résiduelle de cellules béta d'îlots de Langerhans.

3. Utilisation suivant les revendications 1 ou 2, dans lequel l'antagoniste est un anticorps capable de se lier à un polypeptide récepteur d'interféron alpha.

4. Utilisation suivant les revendications 1 ou 2, dans lequel l'antagoniste est un anticorps capable de se lier à une activité biologique d'un interféron alpha et de la neutraliser.

5. Utilisation suivant les revendications 1 ou 2, dans lequel l'antagoniste est un polypeptide récepteur d'interféron alpha.

6. Utilisation suivant la revendication 4, dans lequel l'interféron alpha est choisi parmi IFN-alpha-K, IFN-alpha-5, IFN-alpha-A (IFN-alpha-2), IFN-alpha-D (IFN-alpha-1), IFN-alpha-H1, IFN-alpha-B2, IFN-alpha-B, IFN-alpha-4b, IFN-alpha-C, IFN-alpha-L, IFN-alpha-J1, IFN-alpha-J2, IFN-alpha-I, IFN-alpha-F, IFN-alpha-WA, IFN-alpha-Gx-1, IFN-alpha-76, IFN-alpha-88 et leurs allèles.

7. Utilisation suivant la revendication 4, dans lequel l'anticorps est capable de se lier à plus d'un interféron et de les neutraliser.

8. Utilisation suivant la revendication 4, dans lequel l'activité biologique est choisie parmi une activité anti-prolifération et une activité antivirale.

9. Utilisation suivant la revendication 4, dans lequel l'anticorps est capable de se lier à un interféron alpha synthétisé par une cellule eucaryote et de le neutraliser, mais n'est pratiquement pas capable de neutraliser un interféron alpha de la même sous-classe qui a été synthétisé dans une culture de cellules bactériennes recombinantes.

10. Utilisation suivant l'a revendication 4, dans lequel l'interféron alpha est un interféron alpha de classe I.

11. Utilisation suivant la revendication 4, dans lequel l'interféron alpha est un interféron alpha de classe II.

12. Utilisation suivant la revendication 2, dans lequel l'antagoniste est administré au patient pendant la période de rebond.

13. Utilisation suivant la revendication 2, dans lequel l'antagoniste est administré au patient avant le développement d'une insulino-dépendance.
